# EUROPÄISCHE PATENTSCHRIFT

(11) **EP 3 897 694 B1**
(45) Veröffentlichungstag und Bekanntmachung des Hinweises auf die Patenterteilung: **23.04.2025**
(21) Anmeldenummer: 19835661.0
(22) Anmeldetag: 20.12.2019
(51) Int. Cl.: A61K 38/39, A61K 38/04, A61P 17/00, A61P 1/04, A23L 33/18, A61K 8/65, A61Q 1/00, C07K 14/44, C07K 14/78, A61K 31/56, A61K 35/32, A61K 35/36

(54) **KOLLAGENHYDROLYSAT ZUR VERWENDUNG GEGEN NEURODERMITIS**
COLLAGEN HYDROLYSATE FOR USE IN ATOPIC DERMATITIS
HYDROLYSAT DE COLLAGÈNE DESTINÉ À ÊTRE UTILISÉ CONTRE LA DERMATITE ATOPIQUE

(30) Priorität: 21.12.2018 DE 102018133374
(43) Veröffentlichungstag der Anmeldung: 27.10.2021
(73) Patentinhaber: GELITA AG, 69412 Eberbach (DE)
(72) Erfinder: HAUSMANNS, Stephan, 69126 Heidelberg (DE); OESSER, Steffen, 24960 Glücksburg (DE); DOLLE, Franziska, 69412 Eberbach (DE); FRECH, Hans-Ulrich, 69469 Weinheim (DE)
(74) Vertreter: Hoeger, Stellrecht & Partner Patentanwälte mbB
(86) Internationale Anmeldenummer: PCT/EP2019/086839
(87) Internationale Veröffentlichungsnummer: WO 2020/128070

(56) Entgegenhaltungen:
- WO-A1-2020/094728
- DE-A1- 102010 060 564
- DE-A1- 102012 101 911
- RO-B1- 115 690
- DATABASE WPI Week 201531, Derwent World Patents Index; AN 2015-25020D, XP002798648

## Beschreibung

Die vorliegende Erfindung betrifft Kollagenhydrolysat zur Verwendung als Wirkstoff bei der Behandlung von Neurodermitis.

Die Neurodermitis, die auch als atopisches Ekzem bezeichnet wird, ist eine chronische Hautkrankheit, von der aktuell in den Industriestaaten 1 bis 3% der Erwachsenen betroffen sind und 5 bis 20% der Kinder. Dabei nimmt die Häufigkeit der Neurodermitis generell zu, wobei seit der Mitte des 20. Jahrhunderts von einem vier- bis sechsmal häufigeren Auftreten der Krankheit ausgegangen wird. Auch wenn die genaue Krankheitsursache noch nicht vollständig geklärt ist, handelt es sich nach aktuellem Stand der Forschung um eine Autoimmunerkrankung, bei der sowohl eine genetische Veranlagung als auch Umwelteinflüsse eine Rolle spielen.

Allgemein äußert sich die Neurodermitis in einer gestörten Barrierefunktion der Haut, wobei als konkrete Symptome vor allem gerötete, schuppende, manchmal auch nässende Ekzeme auftreten. Diese sind verbunden mit einem akuten oder chronischen Juckreiz, der sehr stark sein kann und oft auch nachts anhält. Insbesondere der Juckreiz kann zu einer erheblichen Beeinträchtigung der Lebensqualität der Betroffenen führen. Eine Bewertung des Schweregrades der Neurodermitis kann nach dem SCORAD-Index erfolgen (Scoring Atopic Dermatitis).

Eine vollständige Heilung der Neurodermitis ist bisher nicht möglich, zur Behandlung der Symptome stehen jedoch verschiedene Ansätze zur Verfügung. Im Mittelpunkt steht dabei die Basispflege der Haut in Form einer topischen Behandlung mit Salben, Cremes oder Lotionen, um die Barrierefunktion zu stabilisieren und die Empfindlichkeit der Haut gegenüber Irritationen und dem Eindringen von Allergenen abzuschwächen. Sofern diese Maßnahme bei einer stärkeren Ausprägung der Krankheit bzw. der Symptome nicht mehr ausreicht, werden als therapeutische Maßnahme insbesondere Corticosteroide als entzündungshemmende Mittel eingesetzt. Diese Präparate mit Cortisolwirkung werden umgangssprachlich häufig als Cortison bezeichnet. Deren Anwendung erfolgt meistens topisch auf den betroffenen Hautstellen, wobei aufgrund der bekannten Nebenwirkungen von Corticosteroiden, insbesondere auch auf die Haut (wie z.B. Hautatrophie, Pigmentstörungen und Ekchymosen), die Anwendung nur kurzzeitig erfolgen sollte.

Die DE 10 2012 101911 A1 offenbart die Verwendung von Kollagenhydrolysat zur Behandlung und/oder Vorbeugung von Cellulite.

Die RO 115 690 B1 offenbart eine Salbe gegen Akne und Seborrhö, die 1 bis 3% Kollagenhydrolysat umfasst.

Die CN 104 382 943 A offenbart die Verwendung eines Kollagenpeptide enthaltenden Extraktes aus Fischschuppen zur Behandlung und/oder Vorbeugung von Schuppenflechte.

Es besteht daher ein großer Bedarf an Wirkstoffen zur Behandlung von Neurodermitis und anderen Erkrankungen, die mit weniger Nebenwirkungen einhergehen und die alternativ oder ergänzend zu Corticosteroiden eingesetzt werden können.

Zur Lösung dieser Aufgabe wird gemäß der vorliegenden Erfindung die Verwendung von Kollagenhydrolysat als Wirkstoff bei der Behandlung von Neurodermitis vorgeschlagen. Eine entsprechende Wirksamkeit von Kollagenhydrolysat ergibt sich insbesondere aus einer klinischen Studie, bei der Kollagenhydrolysat bzw. ein Placebo an Patienten mit Neurodermitis verabreicht wurde.

Verschiedene vorteilhafte Wirkungen von Kollagenhydrolysat sind bereits seit längerer Zeit bekannt, insbesondere die Anwendung von Kollagenhydrolysat bei Osteoporose oder bei Gelenkbeschwerden. Auch positive Wirkungen von Kollagenhydrolysat auf die Gesundheit der Haut wurden bereits beschrieben, z.B. in der internationalen Patentanmeldung WO 2012/065782 A2. Dennoch ist die Wirksamkeit von Kollagenhydrolysat bei der Behandlung von Neurodermitis überraschend.

Kollagenhydrolysat ist als ein Abbauprodukt von tierischen Ausgangsmaterialien, die auch als Lebensmittel verwendet werden, ein gesundheitlich völlig unbedenkliches Produkt, von dem keine schädlichen Nebenwirkungen bekannt sind. Es bedarf keiner rechtlichen Zulassung als Arzneimittel, sondern kann insbesondere in Form eines Nahrungsergänzungsmittels in Verkehr gebracht und verwendet werden. Im Rahmen der vorliegenden Erfindung ist sowohl eine Verwendung von Kollagenhydrolysat als Nahrungsergänzungsmittel, als rezeptfreies Arzneimittel (OTC) oder als verschreibungspflichtiges Arzneimittel (insbesondere in Kombination mit anderen Wirkstoffen) umfasst.

Das Kollagenhydrolysat wird günstigerweise oral verabreicht. Es ist bekannt, dass die Peptide des Kollagenhydrolysats selbst bei relativ hohen Molekulargewichten von bis zu 10.000 Da im Darm mindestens zu einem gewissen Anteil resorbiert werden. Die bevorzugte Menge an oral verabreichtem Kollagenhydrolysat liegt bei einer täglichen Dosis von 2 bis 15 g, weiter bevorzugt von 3 bis 10 g. Eine positive Wirkung bei Neurodermitis konnte z.B. mit einer täglichen Dosis von 5 g Kollagenhydrolysat gezeigt werden.

Bei einer bevorzugten Ausführungsform der Erfindung wird das Kollagenhydrolysat zur unterstützenden Behandlung neben einer Behandlung der Hautkrankheit mit einem Corticosteroid verwendet. Auch wenn auf ein Corticosteroid häufig nicht vollständig verzichtet werden kann, um eine ausreichende Linderung der Symptome herbeizuführen, ermöglicht die Verabreichung von Kollagenhydrolysat doch zumindest eine Verringerung der verabreichten Menge an Corticosteroid, was im Hinblick auf dessen Nebenwirkungen bereits eine deutliche Verbesserung für die Betroffenen darstellt.

Das Corticosteroid, neben dem das Kollagenhydrolysat erfindungsgemäß verwendet werden kann, wird in der Regel topisch verabreicht, insbesondere in Form einer Salbe. Alternativ ist auch eine orale Verabreichung von Corticosteroiden möglich. In diesem letzteren Fall kann gemäß einer weiteren Ausführungsform der Erfindung eine Zusammensetzung verabreicht werden, die das Kollagenhydrolysat und das Corticosteroid enthält, d.h. in Form eines Kombinationspräparates.

Das Corticosteroid ist vorzugsweise ein Glucocorticoid, wie z.B. Hydrocortison. Es sind eine Vielzahl an Glucocorticoiden bekannt, die in vier Klassen von "schwach wirksam" (Klasse I) bis "sehr stark wirksam" (Klasse IV) eingeteilt werden.

Ein weiterer Aspekt der Offenbarung betrifft Kollagenhydrolysat zur Verwendung als Wirkstoff zur Milderung von Nebenwirkungen von Corticosteroiden, insbesondere von deren Nebenwirkungen auf die Haut, wie z.B. Hautatrophie, Pigmentstörungen und Ekchymosen. Entsprechende Hinweise ergeben sich ebenfalls aus der bereits erwähnten klinischen Studie, auf die weiter unten im Detail eingegangen wird.

Die oben genannten Nebenwirkungen von Corticosteroiden (insbesondere von Glucocorticoiden) sind zumindest teilweise durch eine Verminderung der Kollagenbiosynthese durch die Hautzellen (Fibroblasten) bedingt (siehe z.B. Oikarinen et al., Journal of Investigative Dermatology 98 (1992) 220-225). Zellversuche *in vitro* haben überraschenderweise gezeigt (siehe unten), dass diese Verminderung der Kollagenbiosynthese durch die Anwesenheit von Kollagenhydrolysat fast vollständig kompensiert werden kann.

Das Kollagenhydrolysat zur Verwendung gemäß der Erfindung weist typischerweise ein mittleres Molekulargewicht von 500 bis 15.000 Da auf, bevorzugt von 1.000 bis 8.000 Da, weiter bevorzugt von 1.500 bis 5.000 Da, am meisten bevorzugt von 1.800 bis 2.200 Da. Mit diesen Angaben ist stets das gewichtsmittlere Molekulargewicht gemeint, welches insbesondere durch Gelpermeationschromatographie bestimmt werden kann.

Das Kollagenhydrolysat ist vorzugsweise durch enzymatische Hydrolyse eines kollagenhaltigen Ausgansmaterials hergestellt. Für diese Hydrolyse werden insbesondere Endopeptidasen oder Exopeptidasen mikrobiellen oder pflanzlichen Ursprungs eingesetzt. Durch geeignete Auswahl der Peptidasen und der Hydrolysebedingungen können Kollagenhydrolysate in dem jeweils gewünschten Molekulargewichtsbereich hergestellt werden.

Das kollagenhaltige Ausgangsmaterial ist in der Regel ausgewählt aus Haut oder Knochen von Wirbeltieren, bevorzugt von Säugetieren oder Vögeln, und insbesondere aus der Haut von Rindern oder Schweinen (Rinderspalt bzw. Schweineschwarte). Alternativ kann das kollagenhaltige Ausgangsmaterial ausgewählt sein aus Haut, Knochen und/oder Schuppen von Fischen, insbesondere Kalt- oder Warmwasserfischen.

Das Kollagenhydrolysat kann entweder in einem einstufigen Verfahren aus diesen Ausgangsmaterialien hergestellt sein oder über die Zwischenstufe Gelatine, wobei in diesem Fall sowohl Gelatine vom Typ A als auch vom Typ B verwendet werden kann.

Vorzugsweise ist das Kollagenhydrolysat durch die nacheinander folgende Einwirkung von mindestens zwei Endoproteasen mit einer unterschiedlichen Spezifität, insbesondere von mindestens zwei verschiedenen Metalloproteasen und/oder Serinproteasen, hergestellt, d.h. von Proteasen, die die Aminosäuresequenz der Kollagenmoleküle jeweils vor bzw. hinter bestimmten Aminosäuren spalten. Günstigerweise handelt es sich bei den Metalloproteasen und/oder Serinproteasen um Enzyme aus den Mikroorganismen *Bacillus subtilis, Bacillus licheniformis, Bacillus amyloliquefaciens, Aspergillus oryzae* und *Aspergillus melleus.*

Durch die Auswahl geeigneter Endoproteasen kann nicht nur eine bestimmte Molekulargewichtsverteilung des Kollagenhydrolysats erhalten werden, sondern es wird auch die Art der Aminosäuren an den Termini der in dem Hydrolysat enthaltenen Peptide beeinflusst. In dieser Hinsicht ist es z.B. bevorzugt, wenn mindestens 50% der N-terminalen Aminosäuren des Kollagenhydrolysats hydrophobe Aminosäuren sind, insbesondere Alanin, Leucin und Isoleucin.

Alternativ zur enzymatischen Hydrolyse kann das Kollagenhydrolysat im Rahmen der Erfindung durch rekombinante Genexpression hergestellt sein. Durch den Einsatz von natürlichen Kollagensequenzen, insbesondere aus Rindern oder Schweinen, und deren Expression in gentechnisch modifizierten Zellen (z.B. Hefen, Bakterien oder Pflanzenzellen, insbesondere Tabak) können Produkte hergestellt werden, die mit den Hydrolyseprodukten der entsprechenden kollagenhaltigen Rohstoffe im Wesentlichen identisch sind. Dabei ist es möglich, eine engere bzw. exakt vorgegebene Molekulargewichtsverteilung zu erhalten.

Die Wirksamkeit von Kollagenhydrolysat bei der Behandlung von Neurodermitis wird anhand der nachfolgend beschriebenen klinischen Studie näher erläutert.

Ferner werden Zellversuche *in vitro* beschrieben, welche die Wirksamkeit von Kollagenhydrolysat bei der Milderung der Nebenwirkungen von Corticosteroiden belegen.

In den Figuren zeigen:
- Figur 1:: Ein Säulendiagramm zum Verbrauch von Corticosteroid und Hautpflegesalbe während des Studienzeitraums;
- Figur 2:: ein Säulendiagramm zur Entwicklung des pH-Wertes der Haut während des Studienzeitraums;
- Figur 3:: ein Säulendiagramm zur Veränderung des Index "Beeinträchtigung von Hausarbeit/Erledigungen durch Juckreiz" während des Studienzeitraums;
- Figur 4:: Säulendiagramme zur Biosynthese von Kollagen Typ I und Proteoglykanen durch Fibroblasten;
- Figur 5:: Säulendiagramme zur Biosynthese von Kollagen Typ I und Proteoglykanen durch Chondrocyten; und
- Figur 6:: Säulendiagramme zur Biosynthese von Kollagen Typ I und Proteoglykanen durch Osteoblasten.

### 1. Klinische Studie

### 1.1 Studiendesian

Die klinische Studie wurde durchgeführt mit dreißig Probanden (je 15 Männer und Frauen) mit der Diagnose Neurodermitis, wobei der Schweregrad der Neurodermitis gemäß dem SCORAD-Index bei allen Probanden im Bereich von 15 bis 40 lag. Es handelt sich um eine randomisierte, doppelblinde, placebokontrollierte Studie, d.h. die Probanden wurden zufällig in eine Behandlungsgruppe und eine Placebogruppe mit je 15 Personen eingeteilt, wobei weder die Probanden noch das behandelnde Personal über die Einteilung der einzelnen Probanden informiert waren.

Die Probanden der Behandlungsgruppe erhielten während des gesamten Studienzeitraums von 12 Wochen eine tägliche Dosis von 5 g Kollagenhydrolysat. Hierbei handelt es sich um das von der Anmelderin vertriebene Produkt Verisol B, ein durch enzymatische Hydrolyse von Rinderkollagen hergestelltes Kollagenhydrolysat mit einem mittleren Molekulargewicht von ca. 2.000 Da. Das Herstellungsverfahren von Verisol B entspricht im Wesentlichen dem in der WO 2012/065782 A2 beschriebenen Herstellungsverfahren.

Die Probanden der Placebogruppe erhielten statt des Kollagenhydrolysats eine tägliche Dosis von 5 g Maltodextrin, wobei das Kollagenhydrolysat und das Placebo hinsichtlich Verpackung, Textur und Geschmack nicht zu unterscheiden waren.

Während der Studie war es den Probanden freigestellt, ob und in welchem Umfang sie zur Linderung der Beschwerden eine Hautpflegesalbe (Ungentum leniens) oder eine Corticosteroid enthaltende Salbe (Triamcinolonacetonid) anwenden. Dabei wurden die verbrauchten Mengen an Hautpflegesalbe und Corticosteroid für jeden einzelnen Probanden während des Studienzeitraums erfasst.

### 1.2 Verbrauch von Hautpflegesalbe und Corticosteroid

Ein bemerkenswertes Ergebnis der Studie ist, dass der Verbrauch sowohl von Hautpflegesalbe als auch von Corticosteroid enthaltender Salbe in der Untersuchungsgruppe signifikant geringer ist als in der Placebogruppe. Dies gilt sowohl für das erste Drittel (4 Wochen) des Studienzeitraums als auch für den Zeitraum von der 5. bis zur 12. Woche, und jeweils besonders ausgeprägt für den Verbrauch an Corticosteroid.

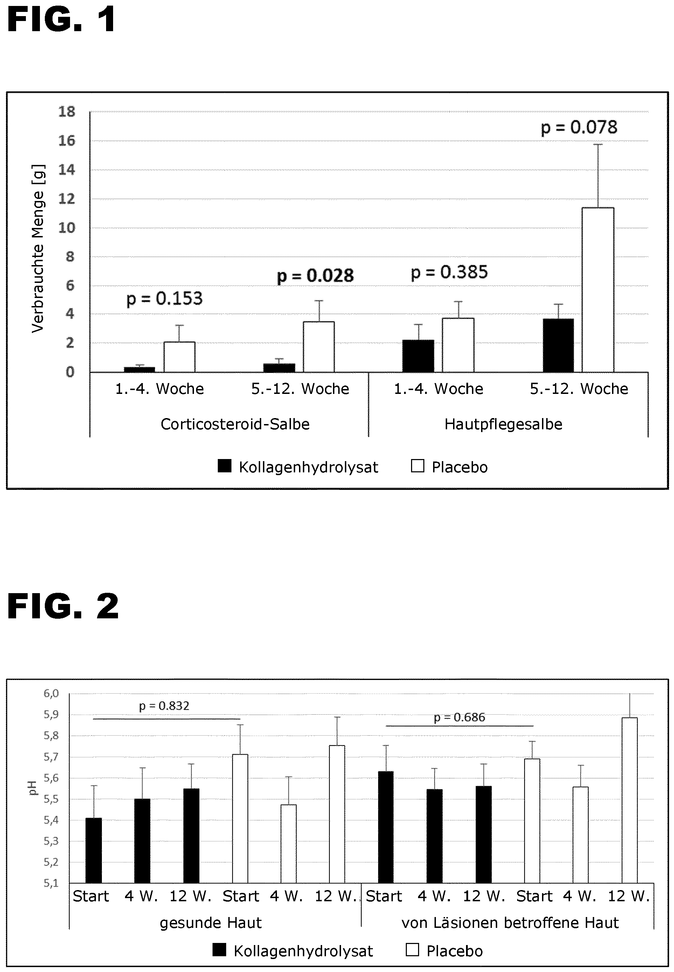

In der Figur 1 sind die entsprechenden Ergebnisse als Säulendiagramm dargestellt, d.h. der durchschnittliche Verbrauch von Corticosteroid enthaltender Salbe und Hautpflegesalbe in Gramm in den beiden Gruppen für die jeweiligen Zeiträume.

Dieses Ergebnis belegt die Wirksamkeit von Kollagenhydrolysat bei der Behandlung von Neurodermitis. Da die Menge an Corticosteroid und Hautpflegesalbe von den Probanden je nach Bedarf frei gewählt werden konnte, kann die geringere benötigte Menge in der Untersuchungsgruppe nur dadurch erklärt werden, dass ein Teil der Symptome unmittelbar durch das oral verabreichte Kollagenhydrolysat gelindert werden konnte.

### 1.3 Messung des pH-Wertes der Haut

Der pH-Wert der Haut, der ein Indikator für deren Barrierefunktion ist, wurde bei allen Probanden zu Beginn der Studie, nach vier Wochen und nach zwölf Wochen mit einem Skin-pH-Meter (Courage + Khazaka electronic GmbH) gemessen. Die Messungen erfolgten im Bereich des Oberarms, und zwar sowohl an gesunden als auch von Läsionen betroffenen Hautstellen.

Die Ergebnisse sind in der Figur 2 als Säulendiagramm dargestellt.

Bei der Behandlungsgruppe nähert sich der pH-Wert von Hautstellen mit Läsionen während des Studienzeitraums tendenziell dem pH-Wert von gesunder Haut im Bereich von 5,3 bis 5,6 an. Demgegenüber ist in der Placebogruppe der pH-Wert sowohl der gesunden Haut als auch der Hautstellen mit Läsionen nach zwölf Wochen abnormal erhöht.

Dieses Ergebnis lässt den Schluss zu, dass die orale Verabreichung von Kollagenhydrolysat mindestens teilweise den Nebenwirkungen von Corticosteroiden auf die Haut entgegenwirkt.

### 1.4 Transepidermaler Wasserverlust und Hautfeuchtigkeit

Es wurden während des Studienzeitraums ferner Messungen des transepidermalen Wasserverlustes (TEWL) mit einem Tewameter und der Hautfeuchtigkeit mit einem Corneometer (jeweils Courage + Khazaka electronic GmbH) durchgeführt. Beide Parameter sind, wie der pH-Wert, Indikatoren für die Barrierefunktion der Haut.

Im Ergebnis fanden sich bei diesen beiden Parametern keine signifikanten Unterschiede zwischen der Behandlungsgruppe und der Placebogruppe, weder an gesunden Hautstellen noch an von Läsionen betroffenen Hautstellen. Indirekt lässt dies den Schluss zu, dass trotz der höheren Menge an eingesetztem Corticosteroid in der Placebogruppe und deren Nebenwirkungen dort keine Verschlechterung der Parameter eingetreten ist, was auf die Wirkung des Kollagenhydrolysats zurückzuführen sein könnte.

### 1.5 Bewertung des Juckreizes

Während des Studienzeitraums wurde von den Probanden eine Bewertung des Juckreizes nach einem als "5-D-Pruritus" bezeichneten Schema durchgeführt. Nach diesem Schema wird einerseits der Juckreiz als solcher quantitativ und qualitativ bewertet (Dauer, Ausmaß und Tendenz), die Lokalisation am Körper (Verteilung), sowie die Beeinträchtigung folgender Lebensbereiche durch den Juckreiz: Schlafen; Freizeit/soziale Aktivitäten; Hausarbeit/Erledigungen; Arbeit/Schule.

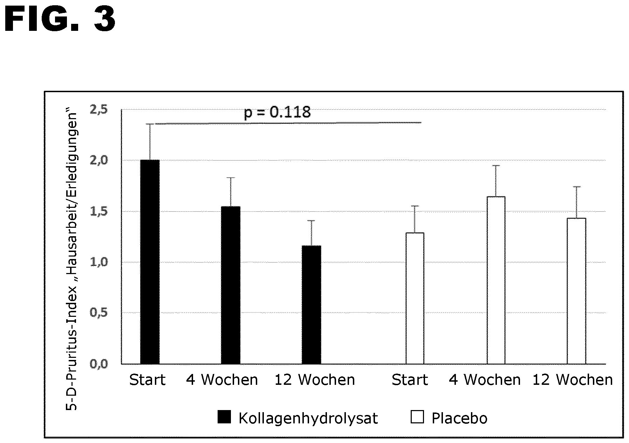

Bei dem Bewertungskriterium "Beeinträchtigung von Hausarbeit/Erledigungen" zeigte sich in der Behandlungsgruppe eine signifikante Verbesserung des Index während des Studienzeitraums im Gegensatz zur Placebogruppe. Die entsprechenden Ergebnisse sind in dem Säulendiagramm der Figur 3 dargestellt (Mittelwerte des Index zu Beginn der Studie nach vier Wochen und nach zwölf Wochen). Dieses Ergebnis spricht unmittelbar für eine Wirksamkeit des Kollagenhydrolysats bei der Linderung des Juckreizes.

Bei den übrigen Bewertungskriterien ergaben sich keine signifikanten Unterschiede in den jeweiligen Indices zwischen der Untersuchungsgruppe und der Placebogruppe. Unter Berücksichtigung der Tatsache, dass in der Placebogruppe deutlich mehr Corticosteroid verbraucht wurde, lässt aber auch dieser Befund indirekt den Schluss zu, dass die Wirkung des Corticosteroids zumindest teilweise durch Kollagenhydrolysat ersetzt werden konnte.

### 2. Zellversuche in vitro

### 2.1 Zellversuche mit Fibroblasten

Eine topische Behandlung mit Corticosteroiden ist insbesondere bei längerer Behandlungsdauer mit negativen Nebenwirkungen wie z.B. Hautatrophie verbunden. Eine wesentliche Ursache für diese Nebenwirkungen ist die Verminderung der Biosynthese von Kollagen, sowie von anderen wichtigen Matrixproteinen wie Proteoglykanen, durch die Fibroblasten.

Durch Zellversuche mit humanen Fibroblasten wurde untersucht, ob Kollagenhydrolysat in Kombination mit Glucocorticoiden einen Einfluss auf die Biosynthese der Matrixproteine hat. Für diese Versuche wurde dasselbe Kollagenhydrolysat (Verisol B) verwendet wie in der oben beschriebenen klinischen Studie.

Primäre humane Fibroblasten wurden in HAM's-F12-Medium kultiviert, welches supplementiert war mit 10% fetalem Kälberserum, 20 U/ml Penicillin-Streptomycin, 50 µg/ml Patricin, 0,05 mg/ml Ascorbinsäure und 0,15 mg/ml Glutamin. Nach Erreichen einer Konfluenz der Zellen von 80% wurde bei drei verschiedenen Ansätzen das Kulturmedium durch frisches Medium ersetzt, welches mit 0,5 mg/ml Kollagenhydrolysat, mit 0,05 mg/ml eines Glucocorticoids (Dexamethason) oder mit einer Kombination beider Komponenten supplementiert war. Ein Kontrollansatz wurde in dem Kulturmedium ohne Zusätze weiter kultiviert.

Nach einer weiteren Kultivierung von sieben Tage wurde die Menge der von den Fibroblasten synthetisierten extrazellulären Matrixproteine ermittelt, wobei die Bestimmung von Kollagen Typ I mit dem "Sircol Soluble Collagen Assay" und die Bestimmung von Proteoglykanen mit dem "Glycosaminoglycan Assay Blyscan" erfolgte (Biocolor Ltd, Großbritannien), jeweils nach Vorgabe des Herstellers.

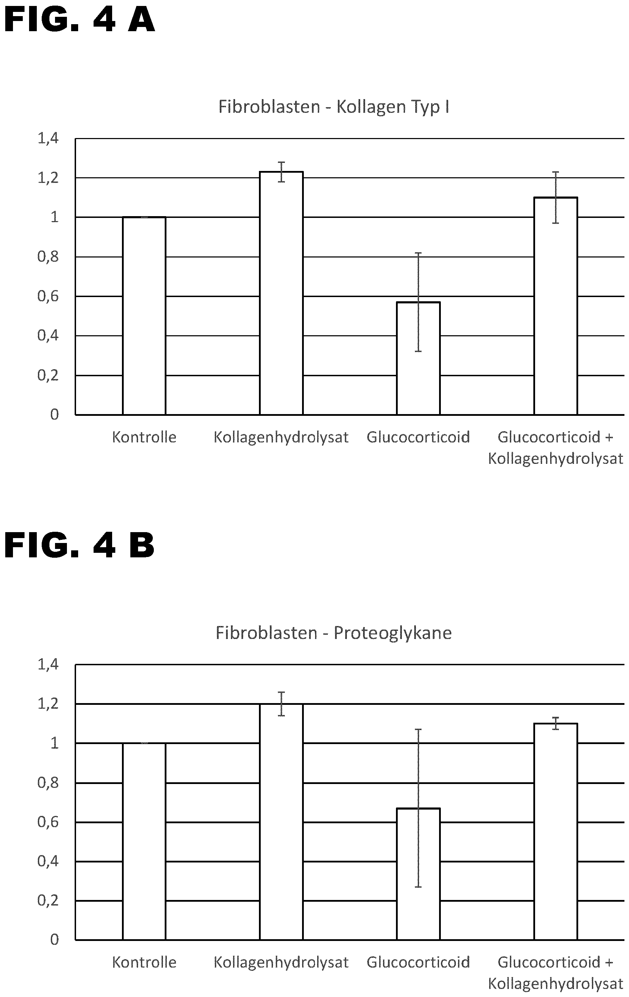

Die Ergebnisse sind in den Säulendiagrammen der Figur 4 dargestellt, wobei Figur 4A die Biosynthese von Kollagen Typ I betrifft und Figur 4B die Biosynthese von Proteoglykanen. Es handelt sich jeweils um die Mittelwerte aus acht Versuchen, angegeben in Relation zum Kontrollansatz. In beiden Fällen zeigt sich zunächst, dass die Biosynthese von Kollagen und Proteoglykanen durch Glucocorticoide erwartungsgemäß stark vermindert ist. Durch zusätzliche Supplementierung mit Kollagenhydrolysat lässt sich diese Verminderung jedoch vollständig kompensieren. Kollagenhydrolysat alleine hat eine leicht stimulierende Wirkung auf die Biosynthese.

Diese Ergebnisse sind ein weiterer Hinweis darauf, dass die Nebenwirkungen von Corticosteroiden wie z.B. Hautatrophie, die durch eine Verminderung der Biosynthese von Matrixproteinen bei Hautzellen bedingt sind, durch die Verabreichung von Kollagenhydrolysat gemindert werden können.

### 2.2 Zellversuche mit Chondrocyten

Dieselben Zellversuche wurden auch mit humanen Chondrocyten (Knorpelzellen) durchgeführt. Die Kultivierung der Zellen und Bestimmung von Kollagen Typ I und Biproteoglykanen erfolgte wie bei den oben beschriebenen Versuchen mit Fibroblasten, mit dem Unterschied, dass ein Kollagenhydrolysat aus Rinderknochengelatine mit einem mittleren Molekulargewicht von ca. 4.000 Da eingesetzt wurde.

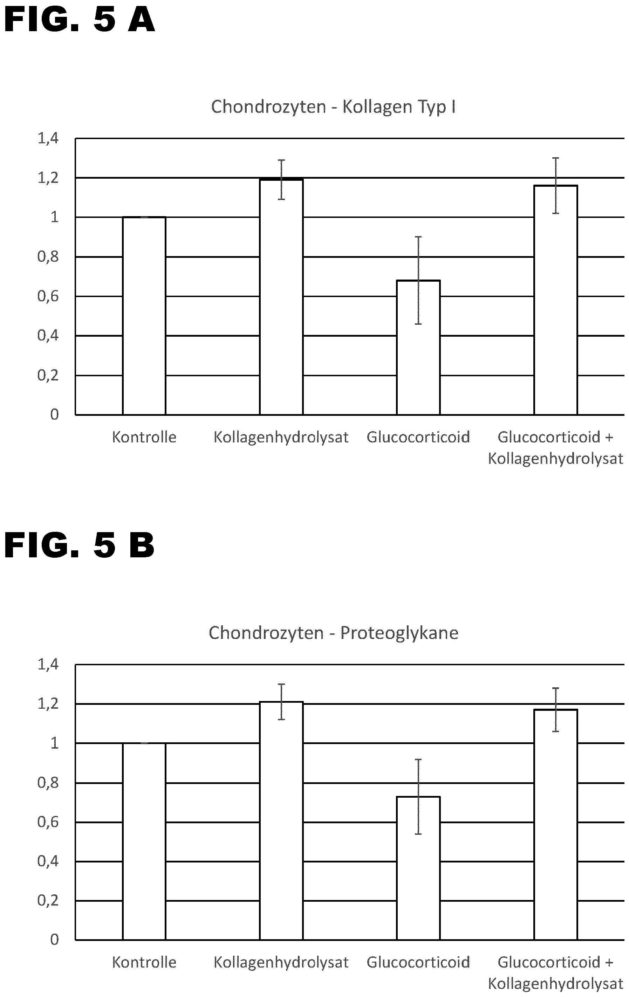

Die Ergebnisse sind in den Säulendiagrammen der Figur 5 dargestellt, wobei Figur 5A die Biosynthese von Kollagen Typ I betrifft und Figur 5B die Biosynthese von Proteoglykanen. Es handelt sich jeweils um die Mittelwerte aus drei Versuchen. Auch hier zeigt sich, dass die durch das Glucocorticoid bedingte Verminderung der Biosynthese durch den Zusatz von Kollagenhydrolysat im Wesentlichen vollständig kompensiert werden kann.

### 2.3 Zellversuche mit Osteoblasten

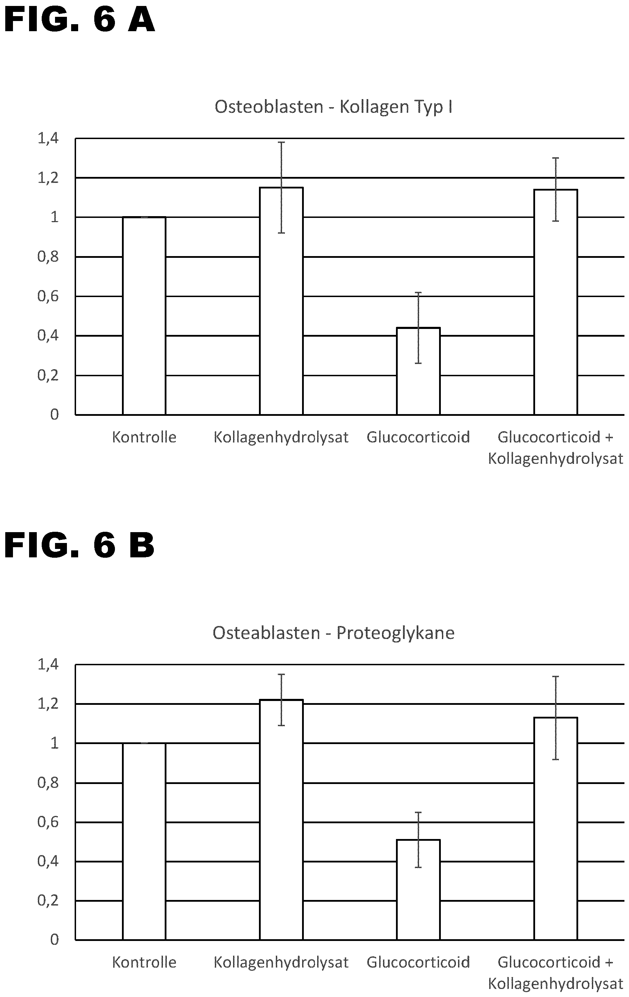

Entsprechende Versuche wurden auch mit humanen Osteoblasten (Knochenzellen) durchgeführt, die Versuchsdurchführung und Bestimmung von Kollagen Typ I und Proteoglykanen erfolgte wie bei den Versuchen mit Chondrocyten (einschließlich des Kollagenhydrolysats mit 4.000 Da). Die Säulendiagramme der Figur 6 zeigen, dass auch im Fall von Osteoblasten die Verminderung der Biosynthese von Kollagen Typ I (Figur 6A) und von Proteoglykanen (Figur 6B) durch das Glucocorticoid im Wesentlichen vollständig durch den Zusatz von Kollagenhydrolysat kompensiert werden kann.

Die Ergebnisse mit Chondrocyten und Osteoblasten zeigen, dass sich die vorteilhaften Wirkungen von Kollagenhydrolysat in Kombination mit Corticosteroiden nicht auf die Haut beschränken, sondern dass auch bei Nebenwirkungen in anderen Gewebetypen mit einer Milderung gerechnet werden kann.

## Patentansprüche

1. Kollagenhydrolysat zur Verwendung als Wirkstoff bei der Behandlung von Neurodermitis.

2. Kollagenhydrolysat zur Verwendung nach Anspruch 1, wobei das Kollagenhydrolysat oral verabreicht wird, bevorzugt in einer täglichen Dosis von 2 bis 15 g, weiter bevorzugt von 3 bis 10 g.

3. Kollagenhydrolysat zur Verwendung nach Anspruch 1 oder 2, wobei das Kollagenhydrolysat zur unterstützenden Behandlung neben einer Behandlung der Neurodermitis mit einem Corticosteroid verwendet wird.

4. Kollagenhydrolysat zur Verwendung nach Anspruch 3, wobei die Verabreichung von Kollagenhydrolysat eine Verringerung der verabreichten Menge an Corticosteroid ermöglicht.

5. Kollagenhydrolysat zur Verwendung nach einem der Ansprüche 3 bis 4, wobei das Corticosteroid topisch verabreicht wird, insbesondere in Form einer Salbe, oder wobei das Corticosteroid oral verabreicht wird, insbesondere in Form einer Zusammensetzung, die das Kollagenhydrolysat und das Corticosteroid enthält.

6. Kollagenhydrolysat zur Verwendung nach einem der Ansprüche 3 bis 5, wobei das Corticosteroid ein Glucocorticoid ist, wie z.B. Hydrocortison.

7. Kollagenhydrolysat zur Verwendung nach einem der vorhergehenden Ansprüche, wobei das Kollagenhydrolysat ein mittleres Molekulargewicht von 500 bis 15.000 Da aufweist, bevorzugt von 1.000 bis 8.000 Da, weiter bevorzugt von 1.500 bis 5.000 Da, am meisten bevorzugt von 1.800 bis 2.200 Da.

8. Kollagenhydrolysat zur Verwendung nach einem der vorhergehenden Ansprüche, wobei das Kollagenhydrolysat durch enzymatische Hydrolyse eines kollagenhaltigen Ausgangsmaterials hergestellt ist.

9. Kollagenhydrolysat zur Verwendung nach Anspruch 8, wobei das kollagenhaltige Ausgangsmaterial ausgewählt ist aus Haut oder Knochen von Wirbeltieren, bevorzugt von Säugetieren, Vögeln oder Fischen, insbesondere aus Haut von Rindern oder Schweinen.

10. Kollagenhydrolysat zur Verwendung nach Anspruch 8 oder 9, wobei das Kollagenhydrolysat durch die nacheinander folgende Einwirkung von mindestens zwei Endoproteasen mit einer unterschiedlichen Spezifität, insbesondere von mindestens zwei verschiedenen Metalloproteasen und/oder Serinproteasen, hergestellt ist.

11. Kollagenhydrolysat zur Verwendung nach Anspruch 10, wobei die Metalloproteasen und/ oder Serinproteasen ausgewählt sind aus Enzymen aus den Mikroorganismen *Bacillus subtilis, Bacillus licheniformis, Bacillus amyloliquefaciens, Aspergillus oryzae* und *Aspergillus melleus.*

12. Kollagenhydrolysat zur Verwendung nach einem der vorangehenden Ansprüche, wobei mindestens 50% der N-terminalen Aminosäuren des Kollagenhydrolysats hydrophobe Aminosäuren sind, insbesondere Alanin, Leucin und Isoleucin.

13. Kollagenhydrolysat zur Verwendung nach einem der Ansprüche 1 bis 7, wobei das Kollagenhydrolysat durch rekombinante Genexpression hergestellt ist.

## Claims

1. A collagen hydrolysate for use as an active substance in the treatment of neurodermatitis.

2. A collagen hydrolysate for use according to claim 1, wherein the collagen hydrolysate is administered orally, preferably in a daily dose of from 2 to 15 g, more preferably from 3 to 10 g.

3. A collagen hydrolysate for use according to claim 1 or 2, wherein the collagen hydrolysate is used for supportive treatment in addition to a treatment of the neurodermatitis.

4. A collagen hydrolysate for use according to claim 3, wherein the administration of collagen hydrolysate allows a reduction of the administered amount of corticosteroid.

5. A collagen hydrolysate for use according to one of claims 3 to 4, wherein the corticosteroid is administered topically, in particular in the form of an ointment, or wherein the corticosteroid is administered orally, in particular in the form of a composition which contains the collagen hydrolysate and the corticosteroid.

6. A collagen hydrolysate for use according to one of claims 3 to 5, wherein the corticosteroid is a glucocorticoid, for example hydrocortisone.

7. A collagen hydrolysate for use according to one of the preceding claims, wherein the collagen hydrolysate has a mean molecular weight of from 500 to 15,000 Da, preferably from 1,000 to 8,000 Da, more preferably from 1,500 to 5,000 Da, most preferably from 1,800 to 2,200 Da.

8. A collagen hydrolysate for use according to one of the preceding claims, wherein the collagen hydrolysate is produced by enzymatic hydrolysis of a collagen-containing starting material.

9. A collagen hydrolysate for use according to claim 8, wherein the collagen-containing starting material is selected from skin or bone of vertebrates, preferably of mammals, birds or fish, in particular from skin of cattle or pigs.

10. A collagen hydrolysate for use according to claim 8 or 9, wherein the collagen hydrolysate is produced by the successive action of at least two endoproteases having a different specificity, in particular of at least two different metalloproteases and/or serine proteases.

11. A collagen hydrolysate for use according to claim 10, wherein the metalloproteases and/or serine proteases are selected from enzymes from the microorganisms *Bacillus subtilis, Bacillus licheniformis, Bacillus amyloliquefaciens, Aspergillus oryzae* and *Aspergillus melleus.*

12. A collagen hydrolysate for use according to one of the preceding claims, wherein at least 50% of the N-terminal amino acids of the collagen hydrolysate are hydrophobic amino acids, in particular alanine, leucine and isoleucine.

13. A collagen hydrolysate for use according to one of claims 1 to 7, wherein the collagen hydrolysate is produced by recombinant gene expression.

## Revendications

1. Hydrolysat de collagène destiné à être utilisé comme principe actif dans le traitement de la neurodermite.

2. Hydrolysat de collagène destiné à être utilisé selon la revendication 1, dans lequel l'hydrolysat de collagène est administré par voie orale, de préférence à une dose quotidienne de 2 à 15 g, de manière davantage préférée de 3 à 10 g.

3. Hydrolysat de collagène destiné à être utilisé selon la revendication 1 ou 2, dans lequel l'hydrolysat de collagène est utilisé pour un traitement d'appoint en plus d'un traitement de la neurodermite avec un corticostéroïde.

4. Hydrolysat de collagène destiné à être utilisé selon la revendication 3, dans lequel l'administration d'hydrolysat de collagène permet de réduire la quantité de corticostéroïde administrée.

5. Hydrolysat de collagène destiné à être utilisé selon l'une quelconque des revendications 3 à 4, dans lequel le corticostéroïde est administré par voie topique, en particulier sous la forme d'une pommade, ou dans lequel le corticostéroïde est administré par voie orale, en particulier sous la forme d'une composition qui contient l'hydrolysat de collagène et le corticostéroïde.

6. Hydrolysat de collagène destiné à être utilisé selon l'une quelconque des revendications 3 à 5, dans lequel le corticostéroïde est un glucocorticoïde, tel que de l'hydrocortisone.

7. Hydrolysat de collagène destiné à être utilisé selon l'une quelconque des revendications précédentes, dans lequel l'hydrolysat de collagène présente un poids moléculaire moyen de 500 à 15 000 Da, de manière préférée de 1 000 à 8 000 Da, de manière davantage préférée de 1 500 à 5 000 Da, idéalement de manière préférée de 1 800 à 2 200 Da.

8. Hydrolysat de collagène destiné à être utilisé selon l'une quelconque des revendications précédentes, dans lequel l'hydrolysat de collagène est fabriqué par hydrolyse enzymatique d'un matériau de départ contenant du collagène.

9. Hydrolysat de collagène destiné à être utilisé selon la revendication 8, dans lequel le matériau de départ contenant du collagène est choisi parmi la peau ou les os d'animaux vertébrés, de manière préférée de mammifères, d'oiseaux ou de poissons, en particulier parmi la peau de bovins ou de porcs.

10. Hydrolysat de collagène destiné à être utilisé selon la revendication 8 ou 9, dans lequel l'hydrolysat de collagène est fabriqué par l'action successive d'au moins deux endoprotéases avec une spécificité différente, en particulier d'au moins deux métalloprotéases et/ou protéases de sérine différentes.

11. Hydrolysat de collagène destiné à être utilisé selon la revendication 10, dans lequel les métalloprotéases et/ou les protéases de sérine sont choisies parmi les enzymes des micro-organismes *Bacillus subtilis, Bacillus licheniformis, Bacillus amyloliquefaciens, Aspergillus oryzae* et *Aspergillus melleus.*

12. Hydrolysat de collagène destiné à être utilisé selon l'une quelconque des revendications précédentes, dans lequel au moins 50 % des acides aminés N-terminaux de l'hydrolysat de collagène sont des acides aminés hydrophobes, en particulier l'alanine, la leucine et l'isoleucine.

13. Hydrolysat de collagène destiné à être utilisé selon l'une quelconque des revendications 1 à 7, dans lequel l'hydrolysat de collagène est fabriqué par expression génique recombinante.
